# EUROPEAN PATENT SPECIFICATION

(11) **EP 0 427 806 B1**
(45) Date of publication and mention of the grant of the patent: **29.06.1994**
(21) Application number: 90904259.0
(22) Date of filing: 08.03.1990
(51) Int. Cl.: C11C 1/04, C12P 7/64

(54) **FAT HYDROLYSIS PROCESS**
FETTHYDROLYSEVERFAHREN
PROCEDE D'HYDROLYSE DE MATIERE GRASSE

(30) Priority: 08.03.1989 DK 1115/89
(43) Date of publication of application: 22.05.1991
(73) Proprietor: NOVO NORDISK A/S, 2880 Bagsvaerd (DK)
(72) Inventor: HANSEN, Tomas, T., DK-3450 Alleroed (DK); NORMAN, Barrie, E., DK-3520 Farum (DK)
(86) International application number: DK9000064
(87) International publication number: WO9010687

(56) References cited:
- EP-A- 0 232 933
- GB-A- 2 176 480
- Dialog Information Services,File 351,World Patent Index 81-90,Dialog accession no.83-755473/36,Nippon Oils & Fats KK: "Hydrolysis of fat using lipase by introducing fat droplets into aq. lipase soln. from the bottom", JP-A- 58126794, 830728, 8336 (Basic)
- Dialog Information Services,File 351,World Patent Index 81-90,Dialog accession no. 85-015003/03,Nippon Oils & Fats KK: "Oil and fat hydrolysing system avoids oxidn. of the oil and fat without the need for large amts. of energy for stirring and using low amts. of lipase", JP-A- 59210893, 841129, 8503 (Basic)

## Description

### TECHNICAL FIELD

This invention relates to a process for hydrolyzing fat in the presence of a lipase at a water:fat ratio in the range from 1:3 to 3:1 (by weight).

### BACKGROUND ART

Industrial fat splitting, i.e. hydrolysis of fat or oil to glycerol and fatty acid, is traditionally carried out at high temperatures under pressure (e.g. 250°C, 60 atm.). Processes using lipase for the hydrolysis at moderate temperatures (e.g. 40-60°C) have also been described (e.g. JP-A 51-080305, JP-A 58-126794, JP-A 59-210893, GB-A 2,176,480, WO 88/02775). It is known that the lower temperature gives benefits such as less energy consumption and less degradation of labile fatty acids.

In an industrial lipase-catalyzed process, the water:fat ratio will usually be in the range of 1:3 to 3:1 (by weight) since a low water content leads to incomplete hydrolysis, whereas a high water content leads to a very dilute glycerol solution and high cost of recovery. The degree of hydrolysis should be as high as possible, while the amount of lipase should be low and the reaction time should be short due to economy.

It is the object of this invention to provide a fat hydrolysis process using lipase giving an increased degree of hydrolysis with unchanged lipase dosage and reaction time.

### STATEMENT OF THE INVENTION

It has surprisingly been found that the degree of hydrolysis can be improved by addition of a minor amount of certain cyclodextrins.

This finding is particularly surprising in view of JP-A 63-191802, according to which the lipase-catalyzed hydrolysis of fat is suppressed by the presence of cyclodextrin at a water:fat ratio of about 10:1.

Cyclodextrins have also been used together with lipase in biological systems (US 4,626,511, EP 187,653), i.e. at conditions very different from those used in industrial hydrolysis, such as water:oil ratio about 500:1.

Accordingly, the invention provides a process for hydrolyzing fat in the presence of a lipase at a water:fat ratio in the range from 1:3 to 3:1 (by weight), characterized by the presence of α-, β- or gamma-cyclodextrin, which may optionally be substituted.

### DETAILED DESCRIPTION OF INVENTION

### Fat

The process is applicable in general to any kind of fat or oil, i.e. any material having a high content of triglycerides. It may be a liquid at ambient temperature, such as soy bean oil and many other oils, or it may be a high-melting fat, such as beef tallow.

The reaction temperature will usually be chosen so that the fat is liquid but techniques are known for lipase-catalyzed hydrolysis of solid fat below its melting point (JP-A 57-057799).

### Lipase

For reasons of economy, microbial lipases are generally preferred but animal lipases may also be used in the practice of the invention.

If a high-melting fat is to be hydrolyzed, it is usually preferred to hydrolyse the fat above its melting point, and the lipase should then be stable at this temperature.

A positionally non-specific lipase will generally be preferred in order to obtain a high degree of hydrolysis in a short time. Examples are Candida lipases derived e.g. from C. antarctica or C. cylindraceae (C. rugosa) and pancreas lipase.

A positionally specific lipase may also be used. It is known that a high degree of hydrolysis can be obtained due to acyl migration. An example is Humicola lipase derived e.g. from H. lanuginosa.

The lipase may be used in native or immobilized form.

### Cyclodextrin

β-cyclodextrin (optionally substituted) is preferred, particularly methyl-β-cyclodextrin (especially with an average of 1 to 2 methyl groups per glucose unit) or hydroxyethyl-β-cyclodextrin (especially with an average of 0.5-1.5 hydroxyethyl groups per glucose unit).

The amount of cyclodextrin is preferably above 0.1% (by weight) relative to fat, most preferably above 0.5%. A mixture of different cyclodextrins may be used.

### Reaction Conditions

The process of the invention may be carried out batch-wise or continuously. In a typical batch process, fat and water are stirred at the chosen reaction temperature. Lipase and cyclodextrin are added, and stirring continued. When the desired degree of hydrolysis is reached, stirring is stopped, and the two phases are separated. The water phase may be evaporated to obtain suitably concentrated glycerol. The oil phase may be dried and purified, as desired, by conventional techniques.

### EXAMPLES

### Example 1

40 g of soy bean oil and 40 g of deionized water were mixed with 0.4 g of cyclodextrin as indicated below. Lipase of Humicola lanuginosa was added in an amount corresponding to a lipase activity of 1000 LU/g of oil (For preparation of lipase and definition LU Lipase Unit, see EP 258,068). The mixture was incubated with stirring at 55°C for 48 hours, and the degree of hydrolysis was then determined by titration of the oil phase.

Results are given below. For each type of cyclodextrin, the molar substitution (substituent groups per glucose unit) is given:

| Cyclodextrin | MS | % hydrolysis |
|---|---|---|
| None | - | 86.6 |
| β-cyclodextrin | 0 | 93.9 |
| Hydroxypropyl-β-cyclodextrin | 0.6 | 90.5 |
| Hydroxyethyl-β-cyclodextrin | 1.0 | 92.6 |
| Methyl-β-cyclodextrin | 1.8 | 100.2 |

These results show that all the cyclodextrins tested significantly increase the degree of hydrolysis at otherwise unchanged conditions. Methyl-β-cyclodextrin is seen to give the best result.

## Claims

1. A process for hydrolyzing fat in the presence of a lipase at a water:fat ratio in the range from 1:3 to 3:1 (by weight), characterized by the presence of α-, β- or gamma-cyclodextrin, which may optionally be substituted.

2. A process according to Claim 1, wherein the cyclodextrin is β-cyclodextrin, optionally substituted.

3. A process according to Claim 2, wherein the cyclodextrin is methyl-β-cyclodextrin, preferably with a substitution ratio in the range from 1 to 2 methyl groups per glucose unit.

4. A process according to Claim 2, wherein the cyclodextrin is hydroxyethyl-β-cyclodextrin, preferably with a substitution ratio in the range from 0.5 to 1.5 methyl groups per glucose unit.

5. A process according to any preceding claim, wherein the amount of cyclodextrin is above 0.1% (by weight) relative to fat, preferably above 0.5%.

## Patentansprüche

1. Ein Verfahren zum Hydrolysieren von Fett in der Gegenwart einer Lipase bei einem Wasser:Fett-Verhältnis im Bereich von 1:3 bis 3:1 (gewichtsbezogen), gekennzeichnet durch die Gegenwart von α-, β- oder Gamma-Cyclodextrin, das fakultativ substituiert sein kann.

2. Ein Verfahren nach Anspruch 1, wobei das Cyclodextrin β-Cyclodextrin ist, das fakultativ substituiert ist.

3. Ein Verfahren nach Anspruch 2, wobei das Cyclodextrin Methyl-β-cyclodextrin ist, vorzugsweise mit einem Substitutionsverhältnis im Bereich von 1 bis 2 Methylgruppen pro Glucoseeinheit.

4. Ein Verfahren nach Anspruch 2, wobei das Cyclodextrin Hydroxyethyl-β-cyclodextrin ist, vorzugsweise mit einem Substitutionsverhältnis im Bereich von 0,5 bis 1,5 Methylgruppen pro Glucoseeinheit.

5. Ein Verfahren nach einem vorangehenden Anspruch, wobei die Cyclodextrinmenge über 0,1 % (gewichtsbezogen) relativ zu Fett liegt, vorzugsweise über 0,5 %.

## Revendications

1. Procédé pour l'hydrolyse de matières grasses en présence d'une lipase dans un rapport eau:matières grasses dans la plage de 1:3 à 3:1 (en poids), caractérisé par la présence d'α-, β- ou gamma-cyclodextrine pouvant être facultativement substituée.

2. Procédé selon la revendication 1, dans lequel la cyclodextrine est la β-cyclodextrine, facultativement substituée.

3. Procédé selon la revendication 2, dans lequel la cyclodextrine et la méthyle-β-cyclodextrine, de préférence avec un rapport de substitution dans la plage de 1 à 2 groupes méthyle par unité de glucose.

4. Procédé selon la revendication 2, dans lequel la cyclodextrine et l'hydroxyéthyle-β-cyclodextrine, de préférence avec un rapport de substitution dans la plage de 0,5 à 1,5 groupes méthyle par unité de glucose.

5. Procédé selon l'une quelconque des revendications précédentes, dans lequel la quantité de cyclodextrine est au-dessus de 0,1 % (en poids) par rapport à la matière grasse, de préférence au-dessus de 0,5 %.
